# DEMANDE DE BREVET EUROPEEN

(11) **EP 1 375 509 A1**
(43) Date de publication de la demande: **02.01.2004**
(21) Numéro de dépôt: 03290709.9
(22) Date de dépôt: 20.03.2003
(51) Int. Cl.: C07J 75/00, C07J 71/00

(54) **Utilisation d'une sapogénine, ou d'un extrait naturel en contenant, pour le traitement des peaux sèches oligoséborrhéiques**

(30) Priorité: 02.04.2002 FR 0204072
(71) Demandeur: L'OREAL, 75008 Paris (FR)
(72) Inventeur: Rubinstenn, Gilles 295, 75005 Paris (FR); Buan, Bruno, 92110 Clichy (FR)
(74) Mandataire: Renard, Emmanuelle

(57) **Abrégé**

La présente invention concerne l'utilisation d'une composition renfermant au moins une sapogénine choisie parmi la diosgénine et l'hécogénine, ou un extrait végétal en contenant, la composition ou la sapogénine étant destinées au traitement des peaux sèches oligoséborrhéiques ou du cuir chevelu sec.

La composition peut être utilisée à des fins cosmétiques, pour traiter le dessèchement de la peau, en particulier après la ménopause, ou à des fins dermatologiques, pour le traitement des désordres liés aux peaux sèches oligoséborrhéiques, en particulier des dermites.

## Description

La présente invention concerne l'utilisation d'une composition renfermant au moins une sapogénine choisie parmi la diosgénine et l'hécogénine, ou un extrait végétal en contenant, la composition ou la sapogénine étant destinées au traitement des peaux sèches oligoséborrhéiques ou du cuir chevelu sec.

De nombreuses femmes à partir de trente-cinq ans, et plus particulièrement après la ménopause, se plaignent fréquemment du dessèchement de leur peau, et des manifestations d'inconfort ou inesthétiques qui en résultent (desquamation, teint terne, atonie cutanée). Or, ce dessèchement est dû, comme on le sait maintenant, à une diminution de la production de sébum avec l'âge.

Par ailleurs, les enfants dont la fonction sébacée n'est pas encore active présentent souvent des signes de peau sèche.

Le sébum est le produit naturel de la glande sébacée qui, conjointement à la sueur produite par les glandes eccrines ou aprocrines, constitue un hydratant naturel de l'épiderme. Il est constitué essentiellement d'un mélange plus ou moins complexe de lipides. Classiquement, la glande sébacée produit du squalène, des triglycérides, des cires aliphatiques, des cires de cholestérol et éventuellement du cholestérol libre (Stewart, M. E., Semin. Dermatol. 11, 100-105 (1992)). L'action des lipases bactériennes convertit une part variable des triglycérides en acides gras libres.

Le sébocyte constitue la cellule compétente de la glande sébacée. La production de sébum est associée au programme de différenciation terminale de cette cellule. Durant cette différenciation, l'activité métabolique du sébocyte est essentiellement axée sur la biosynthèse des lipides (la lipogénèse) et plus précisément sur la néosyntèse d'acides gras et du squalène.

Un composé permettant de stimuler la production des lipides constituant le sébum, par les cellules de la glande sébacée (les sébocytes), serait donc d'un intérêt certain pour le traitement des peaux sèches oligoséborrhéiques, c'est-à-dire présentant un taux de sébum inférieur à 100 µg/cm² au niveau du front.

A cette fin, il a été proposé dans le brevet US-4,496,556 d'utiliser la DHEA, un stéroïde sécrété par les glandes surrénales, ou ses esters, administrés par voie topique, pour augmenter la production de sébum.

Toutefois, pour des questions réglementaires, il n'est pas toujours possible d'utiliser ce type de composés dans le domaine cosmétique. En outre, son efficacité n'est pas suffisante sur les peaux oligoséborrhéiques. Il subsiste donc le besoin de disposer de composés cosmétiquement acceptables, permettant de stimuler efficacement la fonction sébacée en vue de traiter les peaux sèches oligoséborrhéiques.

La demanderesse a maintenant découvert avec étonnement que certaines sapogénines permettaient de satisfaire ce besoin.

Les sapogénines sont des composés résultant de l'hydrolyse acide (généralement en présence d'acide chlorhydrique ou sulfurique en milieu aqueux bouillant) ou enzymatique (généralement pendant environ 5 jours à environ 37°C), habituellement suivie d'une extraction par un solvant organique apolaire, des saponosides qui sont des hétérosides fonctionnalisés, de poids moléculaire très élevé, présents dans les turbercules de certaines plantes. Comme sources de saponosides, on pourra citer : les plantes de la famille des dioscorées comme *Dioscorea composita, Dioscorea deltoides, Dioscorea floribunda, Dioscorea sylvatica, Dioscorea spiculiflora et Dioscorea villosa,* qui sont riches en dioscine ; et les plantes de la famille des Agaves.

Parmi les sapogénines que l'on peut obtenir à partir des plantes précitées, l'acide médicagénique et les extraits de Medicago en contenant ont déjà été décrits comme utiles dans le traitement des peaux sèches résultant d'un renouvellement cellulaire insuffisant (US-5,273,149). Il n'est toutefois pas suggéré que d'autres sapogénines, telles que la diosgénine et l'hacogénine, puissent avoir un quelconque effet sur les peaux sèches oligoséborrhéiques. Ce type de peau nécessite en effet le recours à des composés stimulant la production de sébum.

Dans le même ordre d'idées, le document FR-2 802 412 divulgue une composition destinée à hydrater notamment la peau, après exposition aux infrarouges. La composition contient, comme actif principal, un extrait placentaire, et également, à titre d'agents antimicrobiens et anti-inflammatoires, des sapogénines dont la ruscogénine et l'hédéragénine. Toutefois, ces compositions ne contiennent pas de diosgénine ni d'hécogénine et elles sont appliquées sur des peaux déshydratées par évaporation de l'eau, due à l'échauffement causé par l'exposition aux infrarouges et non sur des peaux oligoséborrhéiques.

La diosgénine a déjà été décrite comme anti-inflammatoire (Yamada et al., *Am. J. Physiol.,* 273:G355-G364, 1997), comme actif amincissant par son action sur les adipocytes (WO 00/30603), comme inhibiteur de collagénase et comme agent antimicrobien utilisable dans le traitements de diverses pathologies à composante infectieuse dont l'acné et la dermatite séborrhéique (DE-198 41 795).

Il a par ailleurs été décrit par la Demanderesse diverses compositions à base de sapogénines, dont la diosgénine, utilisables en particulier dans le traitement du vieillissement cutané (US2002/0028186 ; US-6,331,535 ; FR-2 811 561 ; FR-2 811 567).

Ainsi, à la connaissance de la Demanderesse, il n'a encore jamais été suggéré d'utiliser la diosgénine ou l'hécogénine dans le traitement des peaux sèches oligoséborrhéiques.

En outre, des extraits de *Dioscorea tokoro,* qui renferment de la dioscine, ont été décrits comme efficaces pour hydrater la peau et ainsi l'assouplir. Ainsi, le document JP-10 194 947 divulgue un extrait de *Dioscorea tokoro* préparé par extraction à l'aide d'eau, d'alcool ou d'acétone à une température comprise entre 0 et 50°C, de préférence de 0°C. Cet extrait est décrit comme utile pour améliorer la souplesse et l'hydratation de la peau en raison des glycoprotéines qu'il contient. Il aurait en outre un effet de suppression de la sécrétion sébacée.

De son côté, le document JP-2000 143 488 divulgue une composition pour prévenir et améliorer le dessèchement cutané, comprenant un extrait de plante choisi parmi une longue liste, dont *Dioscorea tokoro maquino* et *Dioscorea gracillima miq.,* qui sont des sources de saponosides. Là encore, le procédé d'extraction comprend simplement l'immersion de la plante dans un solvant à température ambiante, suivie d'étapes de filtration et de séchage et l'extrait ainsi obtenu peut être considéré comme contenant les saponosides de la plante.

De même, le document WO 00/13693 décrit un complexe végétal anti-pollution, présentant un effet hydratant, qui contient notamment des plantes à saponosides comme agents anti-oxydants. Ces plantes sont extraites par broyage, macération dans un solvant hydroalcoolique à moins de 50°C et filtration.

Ainsi, les procédés d'extraction décrits dans ces documents ne permettent pas d'obtenir des compositions contenant des sapogénines (dont la diosgénine), dans la mesure où ils ne comprennent pas d'étape d'hydrolyse acide ou enzymatique. Les compositions décrites ne contiennent donc que des saponosides (dont la dioscine).

La présente invention a donc pour objet l'utilisation cosmétique d'une sapogénine choisie parmi la diosgénine et l'hécogénine, ou d'un extrait végétal en contenant, en tant qu'agent pour le traitement des peaux sèches oligoséborrhéiques ou du cuir chevelu sec.

Elle a encore pour objet l'utilisation d'une sapogénine choisie parmi la diosgénine et l'hécogénine, ou d'un extrait végétal en contenant, pour la préparation d'une composition, notamment dermatologique, destinée à traiter les désordres liés aux peaux sèches oligoséborrhéiques, en particulier les dermites.

Elle a enfin pour objet un procédé de traitement cosmétique d'une peau sèche ou d'un cuir chevelu sec, comprenant l'application topique sur ladite peau ou ledit cuir chevelu d'au moins une sapogénine choisie parmi la diosgénine et l'hécogénine, ou d'un extrait végétal en contenant, dans un milieu physiologiquement acceptable.

Par "peaux sèches oligoséborrhéiques", on entend au sens de la présente invention des peaux présentant un taux de sébum inférieur à 100 µg/cm² au niveau du front à mi-journée. Ces types de peaux se rencontrent fréquemment après la ménopause, de sorte que la composition selon l'invention est de préférence appliquée sur la peau de femmes ménopausées.

La sapogénine mise en oeuvre selon l'invention peut être choisie parmi la diosgénine et l'hécogénine. La diosgénine est notamment disponible auprès de la société SABINSA sous la dénomination commerciale Diosgenin® .

Par "extraits végétaux", on entend tout extrait végétal renfermant une ou plusieurs sapogénines, après traitement destiné à hydrolyser les saponosides, tel qu'un extrait de Dioscorée, qui contient de la diosgénine, ou un extrait de feuille d'agave contenant de l'hécogénine et de la tigogénine.

Ainsi, la diosgénine peut être extraite des tubercules de certaines Dioscorées, comme indiqué précédemment, par un procédé comprenant successivement : l'hydrolyse à chaud des hétérosides en milieu acide minéral (éventuellement après fermentation et séchage des tubercules) ; et la filtration de la fraction insoluble, qui est ensuite neutralisée, lavée et traitée par un solvant apolaire. D'autres procédés d'extraction sont cependant utilisables.

Les variétés de Dioscorées renfermant de la dioscine, précurseur de diosgénine, sont listées dans la publication de Franklin W. MARTIN, The Species of Dioscorea Containing Sapogenin, Econ. Bot., Vol. 23, 373-384 (1969) à laquelle on pourra se reporter.

Parmi celles-ci, on peut citer les espèces *Dioscorea composita, Dioscorea deltoides, Dioscorea floribunda, Dioscorea sylvatica, Dioscorea spiculiflora et Dioscorea villosa.* L'extrait utilisé selon l'invention est préparé préférentiellement à partir de matériel végétal provenant des espèces *Dioscorea villosa* et *Dioscorea opposita.* Un tel extrait est notamment disponible auprès de la société ACTIVE ORGANICS sous les dénominations commerciales Actigen Y® et Actiphyte Mexican Wild Yam®. Il peut en variante être obtenu auprès de la société OSST sous la dénomination commerciale Wild Yam P.E.® ou auprès de la société ALBAN MÜLLER sous la dénomination commerciale Extrait sec igname sauvage®.

L'hécogénine est disponible auprès de la société SIGMA. Elle peut aussi être obtenue par extraction de feuilles d'Agave de l'espèce *Hechtia texensis,* selon un procédé analogue à celui décrit ci-dessus pour la diosgénine. Un extrait d'Agave peut être obtenu auprès de la Société COSMETOCHEM sous la référence commerciale Herbasol Extract Agave®

La quantité de sapogénine utilisable selon l'invention est bien entendu fonction de l'effet recherché et peut donc varier dans une large mesure.

Pour donner un ordre de grandeur, on peut utiliser la sapogénine, ou l'extrait végétal la contenant, en une quantité représentant de 0,0001% à 5% du poids total de la composition, préférentiellement en une quantité représentant de 0,01% à 1,5% du poids total de la composition.

La composition selon l'invention est généralement adaptée à une application topique sur la peau et/ou le cuir chevelu et elle contient donc un milieu physiologiquement acceptable, c'est-à-dire compatible avec la peau, ses phanères (cils, ongles, cheveux) et/ou les muqueuses.

Pour une application topique sur la peau, cette composition peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau. Elle peut aussi être utilisée comme shampooing ou après-shampooing.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des sapogénines ou des extraits végétaux selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, il sera avantageux d'introduire dans la composition utilisée selon l'invention au moins un composé choisi parmi : les agents desquamants ; les agents hydratants ; les agents anti-inflammatoires ou apaisants ; et les agents stimulant la prolifération et/ou la différenciation des kératinocytes.

En effet, la stimulation de la séborrhée par les sapogénines selon l'invention peut, chez certaines personnes, fournir un terrain de prolifération pour la microflore résidente de l'ostium folliculaire (Propionibacterium acnés en particulier), provoquant ainsi une hydrolyse importante des triglycérides du sébum en acides gras libres et la réduction des insaturations des acides gras poly-insaturés (acide linoléique en particulier). Ces deux phénomènes peuvent concourir à une kératinisation de l'infundibulum et à la formation d'un micro-comédon. Celui-ci peut dégénérer en comédon, bouchant et dilatant le pore de façon inesthétique. A un stade plus avancé, ce bouchon peut diverger vers une lésion acnéique inflammatoire.

L'ajout d'agents desquamants ou régulant la prolifération ou la différenciation des kératinocytes à la composition selon l'invention permettent d'éviter la formation de ces comédons. De même, des agents anti-bactériens ou bactério-statiques permettraient, en modérant la prolifération de la microflore résidente, d'obtenir le même effet.

En outre, les agents hydratants peuvent compléter l'effet obtenu à l'aide des sapogénines selon l'invention, et les agents anti-inflammatoires ou apaisants sont utiles pour améliorer le confort des peaux sèches oligoséborrhéiques.

L'invention sera maintenant illustrée par les exemples non limitatifs suivants. Dans ces exemples, les quantités sont indiquées en pourcentage pondéral.

### EXEMPLES

### Exemple 1 : Mise en évidence de l'activité de la sapogénine sur la lipogénèse

L'hécogénine et la diosgénine, fournis par SIGMA, ont été testées sur un modèle de sébocytes humains immortalisés en culture, issus de la lignée SZ95 décrite dans Zouboulis, C.C., Seltmann, H., Neitzel, H. & Orfanos, C.E., Establishment and Characterization of an Immortalized Human Sebaceous Gland Cell Line, J. Invest. Dermatol., 113, 1011-1020(1999).

Le test a consisté à mesurer la quantité de lipides produite par les sébocytes de la lignée (à confluence), en présence ou non d'agents actifs, dilué dans le DMSO, de telle sorte que la quantité finale de DMSO dans le milieu de culture soit 0,1%. Après 2 jours de traitement, les cellules adhérentes sont traitées par du Rouge de Nile (1µg/ml). Le contenu en lipides est ensuite quantifié par mesure de la fluorescence du colorant (deux couples d'excitation/emission : 485-540nm pour les lipides neutres et 540-620 nm pour les lipides non neutres). Les résultats sont donnés pour les lipides totaux (combinaison des deux mesures).

L'expérience est réalisée en triplicate (produits dosés et témoin) en plaque de 96 puits et renouvelée 3 fois.

Les résultats sont rassemblés dans le Tableau ci-dessous :

| PRODUIT | VARIATION DES LIPIDES (par rapport au témoin) |
|---|---|
| Hécogénine (0,1 µM) | + 5 % |
| Diosgénine (0,1 µM) | + 4 % |

Comme il ressort de ce Tableau, l'hécogénine et la diosgénine induisent une augmentation de la lipogénèse sébocytaire.

En outre, dans le même test, la DHEA, testée à une concentration 100 fois plus importante (10 µM) n'a conduit qu'à une augmentation de 13 % du contenu en lipides du sébum, tout en étant inactive à la dose de 0,1µM. Les sapogénines utilisées selon l'invention sont donc plus efficaces que la DHEA.

### Exemple 2 : Compositions cosmétiques et dermatologiques

Ces compositions sont préparées de manière classique pour l'homme du métier. Les quantités données dans ces exemples sont indiquées en pourcentages pondéraux.

### A. Lotion

| | |
|---|---|
| Diosgénine | 1 % |
| Acide salicylique | 1 % |
| Propylène glycol | 5 % |
| Alcool | 87 % |
| Eau | qsp 100 % |

Cette lotion peut être utilisée le soir pour redynamiser la fonction sébacée.

### B. Crème

| | |
|---|---|
| Hécogénine | 1 % |
| Acide n-octanoyl-5-salicylique | 1 % |
| Méthylparaben | 0.1 % |
| Propylparaben | 0.1 % |
| Lanoline | 5 % |
| Huile de vaseline | 4 % |
| Huile de sésame | 4 % |
| Alcool cétylique | 5 % |
| Monostéarate de glycérol | 2 % |
| Triéthanolamine | 1 % |
| Propylène glycol | 5 % |
| Carbomer 940 | 0.1 % |
| Eau | qsp 100 % |

Cette crème, utilisée en applications bi-quotidiennes, permet de raviver l'éclat des peaux sèches.

### C. Onguent

| | |
|---|---|
| Diosgénine | 1 % |
| Acide salicylique | 1 % |
| Monostéarate de glycérol | 3 % |
| Propylène glycol | 12 % |
| Petrolatum | 82.9 % |
| Eau | qsp 100 % |

### D. Gel

| | |
|---|---|
| Diosgénine | 1 % |
| Acide salicylique | 1 % |
| Hydroxy propyl cellulose | 1 % |
| PPG-12-Buteth-16 | 2 % |
| Triéthanolamine | 0.2 % |
| Propylène glycol | 5 % |
| Alcool | 45 % |
| Carbomer 940 | 0.2 % |
| Eau | qsp 100 % |

### E. Emulsion huile dans eau

| | |
|---|---|
| Diosgénine | 0.3 % |
| Extrait lyophylisé de romarin | 0.2 % |
| Stéarate de glycérol | 2 % |
| Polysorbate 60 | 1 % |
| Acide stéarique | 1.4 % |
| Triéthanolamine | 0.7 % |
| Carbomer | 0.4 % |
| Huile d'olive | 12 % |
| Fraction liquide du beurre de karité | 12 % |
| Octyldodécanol | 6 % |
| Isononanoate d'isononyle | 10 % |
| Antioxydant | 0.05 % |
| Parfum | 0.5 % |
| Conservateur | 0.3 % |
| Eau | qsp 100 % |

### F. Crème

| | |
|---|---|
| Diosgénine | 0.5 % |
| Rétinol | 0.1 % |
| Glycérine | 3 % |
| Gomme de Xanthane | 0.1 % |
| Stéarate de sorbitan oxyéthyléné | 0.9 % |
| PEG-100 stéarate et glycéryl stéarate | 2.1 % |
| Alcool cétylique | 2.60 % |
| Isononanoate d'isononyle | 11 % |
| Octydodécanol | 15 % |
| Butylhydroxytoluène | 0.1 % |
| Octocrylène | 2 % |
| Triéthanolamine | 0.3 % |
| Acétate de tocophérol | 1 % |
| Conservateurs | 0.6 % |
| Eau | qsp 100 % |

### G. Crème

| | |
|---|---|
| Diosgénine | 0.3 % |
| O-octanoyl-6'-D-maltose ^{*} | 2 % |
| Triéthanolamine | 0.3 % |
| PEG-100 stéarate et glycéryl stéarate | 2.5 % |
| PEG-50 stéarate | 2.5 % |
| Alcool cétylique | 1 % |
| Alcool stéarylique | 3 % |
| Isononaoate d'isononyle | 20 % |
| Propylparaben | 0.1 % |
| Carbopol | 0.3 % |
| Eau | qsp 100 % |

| | |
|---|---|
| * obtenu comme décrit dans la demande EP-0 566 438 | |

## Revendications

1. Utilisation cosmétique d'une sapogénine choisie parmi la diosgénine et l'hécogénine, ou d'un extrait végétal en contenant, en tant qu'agent pour le traitement des peaux sèches oligoséborrhéiques ou du cuir chevelu sec.

2. Utilisation d'une sapogénine choisie parmi la diosgénine et l'hécogénine, ou d'un extrait végétal en contenant, pour la préparation d'une composition destinée à traiter les désordres liés aux peaux sèches oligoséborrhéiques.

3. Utilisation selon la revendication 2, **caractérisée en ce que** lesdits désordres sont les dermites.

4. Utilisation selon l'une quelconque des revendications 1 à 3, **caractérisée en ce que** la sapogénine est la diosgénine.

5. Utilisation selon l'une quelconque des revendications précédentes, **caractérisée en ce que** ladite sapogénine représente de 0,1 à 1,5% du poids total de la composition.

6. Utilisation selon l'une quelconque des revendications précédentes, **caractérisée en ce que** la composition est adaptée à une application topique sur la peau.

7. Utilisation selon l'une quelconque des revendications précédentes, **caractérisée en ce que** ladite composition renferme en outre au moins un composé choisi parmi : les agents desquamants ; les agents hydratants ; les agents anti-inflammatoires ou apaisants ; et les agents stimulant la prolifération et/ou la différenciation des kératinocytes.

8. Utilisation selon l'une quelconque des revendications précédentes, **caractérisée en ce que** la composition est sous la forme d'une émulsion huile-dans-eau.

9. Procédé de traitement cosmétique d'une peau sèche ou d'un cuir chevelu sec, comprenant l'application topique sur ladite peau ou ledit cuir chevelu d'au moins une sapogénine choisie parmi la diosgénine et l'hécogénine, ou d'un extrait végétal en contenant, dans un milieu physiologiquement acceptable.

10. Procédé selon la revendication 9, **caractérisé en ce que** ladite composition est appliquée sur la peau de femmes ménopausées.
